# EUROPÄISCHE PATENTANMELDUNG

(11) **EP 0 717 025 A2**
(43) Veröffentlichungstag der Anmeldung: **19.06.1996**
(21) Anmeldenummer: 95119054.5
(22) Anmeldetag: 04.12.1995
(51) Int. Cl.: C07C 45/34, C07C 45/35, B01J 23/56

(54) **Verfahren zur Herstellung von Carbonylverbindungen durch katalysierte Oxidation von Olefinen und in Mikroemulsionen angeordnete Katalysatoren**

(30) Priorität: 15.12.1994 DE 4444738
(71) Anmelder: BAYER AG, 51368 Leverkusen (DE)
(72) Erfinder: Imre, Laszlo, Dr., D-51375 Leverkusen (DE); Schomäcker, Reinhard, Dr., D-51381 Leverkusen (DE); Daun, Judit, D-51379 Leverkusen (DE)

(57) **Zusammenfassung**

Carbonylverbindungen können durch katalysierte Oxidation von Olefinen mit Sauerstoff oder sauerstoffhaltigen Gasen in einer Mikroemulsion (ME) als Reaktionsmedium und Katalysatorträger hergestellt werden. Die in ME angeordneten und hierzu eingesetzten Katalysatoren sind durch einen Gehalt von 0,0001-10 Gew.-% einer Pd-Verbindung und durch einen Gehalt von 0,0005-20 Gew.-% einer Nebengruppenmetall-Verbindung oder eines Chinons, bezogen auf das Gesamtgewicht der in der ME angeordneten Katalysatoren, gekennzeichnet.

## Beschreibung

Die vorliegende Erfindung betrifft die Oxidation von offenkettigen oder cyclischen Olefinen in einer Mikroemulsion als Reaktionsmedium und Katalysatorträger unter Bildung von Verbindungen, die eine Carbonylfunktion enthalten(offenkettige oder cyclische Aldehyde und Ketone) sowie die in Mikroemulsionen angeordneten Katalysatoren.

Ein bekanntes und allgemein anwendbares Verfahren ist die Oxidation von C-C-Doppelbindungen mit Ozon als Oxidationsmittel (Houben-Weyl: Methoden der Organischen Chemie, Stuttgart 1952, Band 7/1, Seite 333 f.). Dieses Verfahren ist oft für die Lösung von chemischen Problemen im Laboratorium herangezogen worden, hat aber keine technische Bedeutung erlangen können, da die Herstellung und Handhabung von Ozon sehr aufwendig ist und der Umgang mit den als Zwischenprodukten erhaltenen hochexplosiven Ozoniden erhebliche Risiken beeinhalten.

Als weitere Oxidationsmittel für die Umsetzung von C-C-Doppelbindungen sind beispielsweise Kaliumpermanganat, Chromsäure, Salpetersäure, Osmiumtetroxid, Wasserstoffperoxid/Osmiumtetraoxid, Bleitetraacetat und Perjodsäure beschrieben worden, die gezielt für die Lösung von Einzelproblemen eingesetzt wurden (Houben-Weyl: Methoden der Organischen Chemie, Stuttgart 1952, Band 7/1, Seite 347, 351 f.).

Alle diese Methoden sind sehr aufwendig, erfordern teure Oxidationsmittel und sind an spezielle strukturelle Voraussetzungen geknüpft.

Für die Oxidation von Olefinen in technischem Maßstab, sowohl in der Gasphase als auch in der Flüssigphase, sind aus der Patentliteratur eine Anzahl von katalytischen Verfahren bekannt geworden.

Bei der Durchführung von Oxidationen von Olefinen in der Gasphase in Gegenwart von festen Katalysatoren werden ganz allgemein ungenügende Selektivitäten erzielt. Solche Verfahren kommen daher für viele Verbindungen nicht in Frage (US 3 946 081 (1976); Ullmann's Encyklopädie der technischen Chemie, 4. Aufl., Band 17, Seite 483 f.).

Bei der Durchführung von Oxidationsreaktionen in der Flüssigphase stellt das Einbringen des Katalysators (meistens anorganische Verbindungen) in das Reaktionsgemisch wegen der geringeren Löslichkeit der anorganischen Verbindungen in den organischen Verbindungen ganz allgemein ein schwerwiegendes Problem dar. Wegen der damit verbundenen geringen Katalysatorkonzentration wird bei relativ hohen Temperaturen gearbeitet, und man erzielt demnach oft nur geringe Umsätze und unbefriedigende Selektivitäten (DE-OS 2 618 055; DE-OS 2 805 402).

Im Wacker-Hoechst-Verfahren wird Ethylen in wäßriger Phase in Gegenwart von Palladiumchlorid und Kupferchlorid bei 90-130°C und einem Druck bis zu 10 bar mit Sauerstoff oder Luft in technischem Maßstab zu Acetaldehyd oxidiert (DE-PS 1 049 845, 1 061 767, 1 080 994; s. auch Winnacker-Küchler, Chemische Technologie, Band 6, 4. Aufl., Seite 66 f. (1982)). Die Durchführung des Prozesses erfolgt entweder einstufig oder zweistufig (die Reoxidation der Katalysatorlösung erfolgt bei dem zweistufigen Verfahren in einer getrennten Stufe). Es ist ein elegantes, weltweit angewandtes Verahren, jedoch ist es grundsätzlich wünschenswert, bei deutlich niedrigeren Temperaturen arbeiten zu können.

In DE-OS 3 305 000 wird ferner die Oxidation von Cyclopenten zu Cyclopentanon in Gegenwart von Wacker-Hoechst-Katalysatoren in alkoholischen Lösungen beschrieben. Die Löslichkeit der Katalysatorsalze, die man in festem Zustand in das Reaktionsgefäß einspeist, ist in organisch-alkoholischen System jedoch sehr gering, ihre Konzentration daher undefiniert.

Demgegenüber wurde nun gefunden, daß die Oxidation von Olefinen unter Bildung von Carbonylverbindungen (offenkettige oder cyclische Aldehyde und Ketone) mit hoher Selektivität durchgeführt werden kann, wenn man die Olefine mit Sauerstoff oder sauerstoffhaltigen Gasen bei niedrigen Temperaturen in Gegenwart von Katalysatoren in Mikroemulsionen als Reaktionsmedien umsetzt.

Die Erfindung betrifft ein Verfahren zur Herstellung von Carbonylverbindungen der Formel 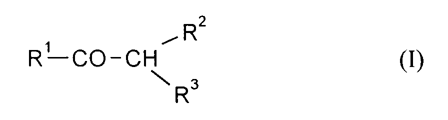
durch katalysierte Oxidation von Olefinen der Formel 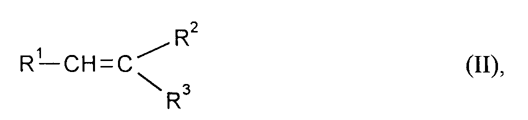
wobei in den Formeln
- R¹, R² und R³: unabhängig voneinander Wasserstoff; geradkettiges oder verzweigtes C₁-C₈-Alkyl, das nicht substituiert oder 1-2fach durch Halogen, Hydroxy, Cyano, COO-C₁-C₄-Alkyl, CO-C₁-C₄-Alkyl, SO₂-O-C₁-C₂-Alkyl oder Phenyl substituiert ist; oder Phenyl oder Naphthyl, die nicht substituiert oder 1-2fach durch Halogen, Hydroxy, Nitro, Cyano, C₁-C₄-Alkyl, COO-C₁-C₄-Alkyl, CO-C₁-C₄-Alkyl oder SO₂-O-C₁-C₄-Alkyl substituiert sind, bedeuten,
und
- R¹ und R³ oder R² und R³: weiterhin gemeinsam eine Alkylenkette (̵CH₂)̵ₘ mit m = 3-10 bilden können und 1-2 der Alkylenketten-C-Atome durch Halogen, Hydroxy, C₁-C₄-Alkyl, COO-C₁-C₄-Alkyl, CO-C₁-C₄-Alkyl, SO₂-O-C₁-C₄-Alkyl oder Phenyl substituiert sein können,
mit Sauerstoff oder sauerstoffhaltigen Gasen, das dadurch gekennzeichnet ist, daß man die Oxidation bei 0-200°C und 0,2-200 bar in Gegenwart eines Katalysatorsystems, bestehend aus einer Palladiumverbindung und einer oder mehrerer Reoxidationsmittel aus der Gruppe von Verbindungen von weiteren Nebengruppenmetallen und Chinonen, in einer Mikroemulsion als Reaktionsmedium und Katalysatorträger durchführt.

In Reaktionsmedien, die aus organischen Komponenten, Wasser und Tensiden bestehen, lassen sich die erfindungsgemäß umzusetzenden Olefine unter Bildung eines einphasigen Systems (Mikroemulsion ME) einbringen. Einphasige ME können auch die in organischen Systemen sonst nicht löslichen, anorganischen Salze als Katalysatoren aufnehmen. Die ME ist somit gleichzeitig Reaktionsmedium und Katalysatorträger.

Die Erfindung betrifft somit weiterhin in ME angeordnete Katalysatoren, insbesondere solche für Reaktionen, bei denen sowohl Edukte als auch Produkte gasförmig sind. Die Produkte und evtl. nicht vollständig umgesetzte Edukte verlassen nach erfolgter Umsetzung den in der ME angeordneten Katalysator, der somit für weitere Reaktionen zur Verfügung steht. Dies ist formal analog zu heterogenen festen Katalysatoren; im Gegensatz zu diesen ist der Kontakt zwischen den in ME angebrachten Katalysatoren und Edukten durch die "Löslichkeit der Edukte in den Katalysatoren" viel intensiver.

Oxidationen in Einphasensystemen weisen Vorteile gegenüber Oxidationen in zwei- oder mehrphasigen Systemen auf. Die ME bildet sich bereits unter leichtem Rühren spontan aus einer Mischung aus Wasser, organischen Verbindungen, beispielsweise dem umzusetzenden Olefin und gegebenenfalls weiteren organischen Lösungsmitteln, und dem Tensid. Die entstehende Dispersion ist optisch transparent und thermodynamisch stabil, d.h. es gibt auch nach langen Zeiten keine Trennung der ME in zwei Phasen (Angewandte Chemie 97, (1985), 655; Ullmann, Vol. 9, Seite 310; Römpp, Chemie Lexikon, S. 2779). Die Tröpfchengröße der ME stellt sich als Funktion der Zusammensetzung im Bereich von 5 bis 100 nm ein und ist unabhängig vom Leistungseintrag, mit dem die Dispergierung erfolgt. Da die Tröpfchengröße weit unterhalb der Wellenlänge des sichtbaren Lichtes liegt, ist die ME optisch transparent wie eine echte Lösung, obwohl Tröpfchen vorliegen wie in einer Emulsion. Der Unterschied zu einer normalen Emulsion ist zum einen in der Größe der Tröpfchen zu sehen (ME: 5 bis 100 nm; sonstige Emulsionen: 0,5 bis 10 µm) und zum anderen in der thermodynamischen Stabilität der ME. Während zur Herstellung einer sonstigen Emulsion in nennenswerten Umfang mechanische Energie eingesetzt werden muß, bildet sich eine ME unter leichtem Rühren spontan aus den Komponenten. Dieser Herstellungsprozeß der ME ist vergleichbar mit dem Mischen zweier mischbarer Flüssigkeiten.

Es wurde gefunden, daß es für die erfindungsgemäß erforderliche Bildung der ME wichtig ist, daß sich die Zusammensetzung und die Temperatur der Mischung innerhalb des Einphasengebietes des Phasendiagrammes des betreffenden Systemes befindet. Für jede ternäre Mischung aus Wasser, Olefin (und gegebenenfalls zusätzlichem organischem Lösungsmittel) und einem Tensid ergibt sich ein eigenes Phasendiagramm. In diesen verschiedenen Phasendiagrammen wird das Einphasengebiet jeweils bei anderen Temperaturen und Zusammensetzungen vorgefunden. Der Temperaturbereich des Einphasengebietes wird durch die Hydrophilie des Tensides bestimmt, d.h. mit zunehmender Hydrophilie zu höheren Temperaturen verschoben. Durch die Verwendung von Tensidmischungen kann der Temperaturbereich des Einphasengebietes gezielt auf eine gewünschte Temperatur eingestellt werden.

Für die Reaktionsgemische wurde das Tensid (bzw. Tensidgemisch) ausgewählt, mit dem das Einphasengebiet sich über die gewünschten Zusammensetzungen und den gewünschten Temperaturbereich (z.B. Raumtemperatur) erstreckt. An das Einphasengebiet grenzen Zweiphasengebiete an, in denen normale, instabile Emulsionen vorliegen.

Geeignet zur Herstellung der ME sind ionische und nichtionische Tenside. Es kann ein einzelnes Tensid oder eine Mischung mehrerer, bevorzugt 1 bis 3 Tenside eingesetzt werden.

Benannt seien im einzelnen: n-Alkyl(C₈-C₁₈)-sulfonate, n-Alkyl(C₈-C₁₈)-benzolsulfonate, n-Alkyl(C₈-C₁₈)-trimethyl-ammoniumsalze, Di-(n-alkyl(C₈-C₁₈))-dimethyl-ammoniumsalze, n-Alkyl-(C₈-C₁₈)-carboxylate, Oligoethylenoxid-EO₂₋₃₀-mono-n-alkylether(C₆-C₁₈), n-Alkyl(C₈-C₁₈)-dimethylaminoxid, n-Alkyl(C₈-C₁₈)-dimethylphosphinoxid oder Oligoethylenoxid-mono-arylether. Die n-Alkylketten können auch durch teilweise ungesättigte Ketten ersetzt sein.

In der ME liegen (i) 1 bis 20 Gew.-% Wasser, (ii) 60 bis 97 Gew.-% an einer oder mehrerer organischer Komponenten aus der Gruppe der umzusetzenden Olefine und organischen Lösungsmitteln und (iii) 2 bis 20 Gew.-% an einem oder mehreren Tensiden vor, alles bezogen auf das Gesamtgewicht der ME.

Als Katalysatoren kommen für das erfindungsgemäße Verfahren eine Palladiumverbindung und als Cokatalysator wenigstens ein Reoxidationsmittel aus der Gruppe von Verbindungen der Übergangselemente der ersten, zweiten und dritten Übergangsreihe, sowie der Aktiniden, wie sie in DE-OS 26 18 055 und Hollemann-Wiberg, Lehrbuch der anorganischen Chemie, 1971, Seite 672 bis 680 beschrieben sind, und von Chinonen in Betracht. Die Reoxidationsmittel bewirken eine Wiederoxidation des Pd-Katalysators, die seinerseits durch die Oxidation des Olefins reduziert worden war; das hierbei reduzierte Reoxidationsmittel wird danach durch Sauerstoff wieder in den oxidierten Zustand übergeführt. Die Chinone können auch in Form der korrespondierenden Hydrochinone eingesetzt werden. Geeignete Chinone sind o- und p-Benzochinon, Naphthochinon, Anthrachinon und andere dem Fachmann bekannte; sie können durch 1 bis 4 Halogenatome (F, Cl, Br), C₁-C₄-Alkylgruppen oder C₁-C₄-Alkoxygruppen, auch durch unterschiedliche von ihnen, substituiert sein. In bevorzugter Weise werden Nebengruppenmetall-Verbindungen, die in verschiedenen Oxidationsstufen vorkommen, als Reoxidationsmittel eingesetzt. Die Reoxidationsmittel werden im folgenden auch als Cokatalysator bezeichnet.

Bevorzugt werden Verbindungen der nachstehend genannten Elemente in Kombination mit Pd als Katalysator verwendet: Eisen, Kobalt, Nickel, Kupfer, Chrom, Zinn, Antimon, Cerium, Rhodium, Platin, Gold.

Als Verbindungen der vorgenannten Übergangselemente, kommen in Frage (DE-OS 2 618 055):
- Salze anorganischer Säuren,
- Salze organischer Carbonsäuren,
- Komplexsalze der Elemente,
- Alkoholate aliphatischer, cycloaliphatischer, araliphatischer und aromatischer Alkohole.

Selbstverständlich können auch Gemische der verschiedenen vorgenannten Cokatalysatoren verwendet werden.

Anstatt der Verbindungen der Übergangselemente als Cokatalysator können auch organische Verbindungen wie z.B. Chinone verwendet werden.

Bevorzugt wird das erfindungsgemäße Verfahren in Gegenwart eines Katalysatorsystems aus Palladiumchlorid oder Palladiumnitrat und Eisennitrat durchgeführt.

Die Konzentration der Salze des Katalysatorsystems in der ME kann in weiten Grenzen variiert werden. Die zugesetzte Menge der Palladiumverbindung kann beispielsweise von 0,0005 bis 10 Gew.-%, vorzugsweise von 0,05 bis 1 Gew.-% der ME betragen. Die Konzentration des Cokatalysators kann unter oder über der Konzentration der Palladiumverbindung liegen, beispielsweise bei 0,0001-20 Gew.-% der Mikroemulsion. Vorteilhaft wird die Konzentration des Cokatalysators höher eingestellt als die der Palladiumverbindung, bevorzugt um einen Faktor 2 bis 100.

Zum Einbringen des Katalysators in eine ME geht man von einer wäßrigen Lösung aus, die mit der ME eine einphasige Lösung bildet. Man kann den Katalysator auch direkt in der ME auflösen.

Als Olefine kommen für das erfindungsgemäße Verfahren beispielsweise solche der obigen Formel (II) mit dem genannten Bedeutungsumfang in Frage.

Bevorzugte Olefine sind solche der Formel 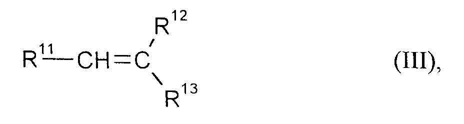
in der
- R¹¹ und R¹²: unabhängig voneinander Wasserstoff; geradkettiges oder verzweigtes C₁-C₄-Alkyl, das nicht substituiert oder durch Halogen, Hydroxy, COO-CH₃, CO-CH₃ oder Phenyl substituiert ist; oder Phenyl, das nicht substituiert oder durch Halogen, Hydroxy, Nitro, CH₃, C₂H₅, COO-CH₃ oder CO-CH₃ substituiert ist, und
- R¹³: Wasserstoff oder Methyl bedeuten; und
- R¹¹ und R¹³ oder R¹² und R¹³: gemeinsam Trimethylen, Tetramethylen oder Pentamethylen bilden können.

Der organische Anteil der ME kann durch das umzusetzende Olefin allein gestellt werden. Es kann jedoch auch ein oxidationsbeständiges organisches, mit Wasser nicht mischbares Lösungsmittel in einer Menge von 10-1000 Gew.-% der Menge des umzusetzenden Olefins mitverwendet werden. Solche Lösungsmitel sind beispielsweise: C₄-C₂₀-Alkane oder C₆-C₁₀-Aromaten, die auch durch Halogen, Nitro oder Estergruppen substituiert sein können und dem Fachmann als typische organische Lösungsmittel geläufig sind.

Im allgemeinen können Temperatur und Druck in dem erfindungsgemäßen Verfahren in weiten Grenzen variiert werden.

So kann das erfindungsgemäße Verfahren in einem Temperaturbereich zwischen 0 und 200°C durchgeführt werden. Vorteilhaft wird im Temperaturbereich von etwa 10 bis 80°C insbesondere bei 15-40°C (Nähe Raumtemperatur) gearbeitet. Die Durchführung der Umsetzung in der Nähe bei Raumtemperatur wird Kaltoxidation genannt.

Es kann bei Normaldruck, vermindertem oder erhöhtem Druck gearbeitet werden; im allgemeinen ist der Druckbereich zwischen 0,2 und 200 bar geeignet. Vorteilhaft wird im Druckbereich von 0,5 bis 100 bar, insbesondere von 1 bis 10 bar gearbeitet.

Als Oxidationsmittel kann im erfindungsgemäßen Verfahren reiner Sauerstoff verwendet werden. Er kann auch als Gemisch mit einem oder mehreren Inertgasen wie Stickstoff, Argon, Kohlendioxid verwendet werden, selbstverständlich auch in Form von Luft.

Die Reaktionszeit kann im erfindungsgemäßen Verfahren in weiten Grenzen variiert werden. Sie kann beispielsweise 0,1 bis 20, vorzugsweise 0,25 bis 10 Stunden betragen und richtet sich u.a. nach der Ansatzgröße.

Zur Durchführung des erfindungsgemäßen Verfahrens kann man die ME zusammen mit dem zu oxidierenden Olefin bilden, beispielsweise mit Cyclopenten oder Cyclohexen, in die dann der gewünschte Katalysator eingebracht wird. Dabei bleibt das ganze System einphasig. Dieses System wird dann mit Sauerstoff oder Sauerstoff enthaltendem Gasgemisch durch feine Verteilung in engen Kontakt gebracht. Dabei kann das erfindungsgemäße Verfahren diskontinuierlich in einfachster Weise in einem Rührautoklaven unter entsprechendem Sauerstoffdruck und geeigneter Temperatur durchgeführt werden, wobei der Sauerstoffdruck mindestens so hoch sein muß, daß die Sauerstoffmenge zur Erzielung des gewünschten Umsatzes ausreichend ist. Im allgemeinen ist jedoch vorteilhafter, auch bei an sich diskontinuierlicher Durchführung des erfindungsgemäßen Verfahrens Sauerstoff kontinuierlich in die flüssige Phase einzuleiten und den Gasstrom mit Hilfe eines Ventilsystems durch Gaszuführung und Gasabführung bei konstantem Druck mit konstanter Strömungsgeschwindigkeit aufrechtzuerhalten (DE-OS 26 18 055).

Zur Erreichung einer hohen Selektivität des gewünschten Reaktionssproduktes kann es vorteilhaft sein, die Reaktion so zu führen, daß nur ein partieller Umsatz erzielt wird; das kann beispielsweise durch entsprechende Wahl der Reaktionsbedingungen, Druck und Temperatur, aber auch der Zusammensetzung der Flüssigphase oder Gasphase, sowie der Katalysatorart und -menge, in bekannter Weise erreicht werden.

Nach Erreichen des gewünschten Umsatzes wird das Reaktionsgemisch aufgearbeitet. Dies kann beispielsweise destillativ erfolgen.

Der große Vorteil der erfindungsgemäßen Arbeitsweise in ME ist, daß man die Aufarbeitung des Reaktionsgemisches zur Gewinnung des Produktes, infolge der chemischen und physikalischen Eigenschaften von ME deutlich einfacher durchführen kann. Vorzugsweise wird die Aufarbeitung wie folgt durchgeführt: Durch eine Erniedrigung der Temperatur gegenüber der Reaktionstemperatur trennt sich die ME in eine erste Phase, in der sich der überwiegende Teil des Tensids und des Wassers (einschließlich des Katalysators) befindet, und in eine zweite Phase, in der das Produkt und nicht umgesetztes Olefin (evtl. mit einem zusätzlichen Lösungsmittel) enthalten sind. Die letztere Phase wird einer Aufarbeitung durch Destillation zugeführt. Die katalysatorhaltige Phase kann mit frischem Edukt versetzt und in die Reaktion zurückgeführt werden. Die Phasentrennung kann bereits teilweise durch die Entstehung der Reaktionsproduktes bei Reaktionstemperatur verursacht werden. Je nach Art des umgesetzten Olefins ist der Einfluß des Produktes auf die Stabilität der ME unterschiedlich groß, so daß unterschiedlich große Temperaturänderungen für die Trennung der ME in die zwei Phasen notwendig sind. Eine solche Trennung kann auch kontinuierlich durchgeführt werden.

Sind die umzusetzenden Olefine und Produkte bei Reaktionstemperatur gasförmig, wird in einer bevorzugten Ausführungsform des erfindungsgemäßen Verfahrens wie folgt gearbeitet: Zunächst wird die eingestellte ME mit dem Katalysator ohne das umzusetzende Olefin in ein geeignetes Reaktionsgefäß, beispielsweise in einen Autoklaven, gefüllt. In diese ME wird dann das Gasgemisch, das sowohl das umzusetzende Olefin als auch das Oxidationsmittel, beispielsweise den Sauerstoff; enthält, kontinuierlich eingeleitet und vermischt. Der Gasstrom kann dabei mit Hilfe eines Ventilsystems zur Gaszuführung und Gasabführung bei konstantem Druck mit konstanter Strömungsgeschwindigkeit aufrechterhalten werden. Durch die Strömungsgeschwindigkeit des Gasgemisches kann eine beliebige Reaktionszeit ("Kontaktzeit") entsprechend dem gewünschten Umsatz eingestellt werden. Das ausströmende Gasgemisch enthält das Reaktionsprodukt, das zur Aufarbeitung abgeführt wird. Die Aufarbeitung beschränkt sich also nur auf das den Reaktor verlassene Gasgemisch, die man in bekannter Weise durchführen kann. Nach dieser Variante des erfindungsgemäßen Verfahrens wird die ME als Katalysatorträger, ähnlich wie ein festes Katalysatorträgermaterial, kontinuierlich über einen längeren Zeitraum verwendet.

Das erfindungsgemäße Verfahren hat gegenüber dem Stand der Technik den Vorteil, daß Olefine in einfacher Weise kontinuierlich oder diskontinuierlich mit hoher Selektivität oxidiert werden können.

Verbindungen, die nach den, erfindungsgemäßen Verfahren hergestellt werden können, nämlich offenkettige oder cyclische Aldehyde und Ketone, werden bekanntlich in einer Vielzahl organischer Synthesen als Zwischenprodukte verwendet.

### Beispiele

### Apparatur

In den nachstehenden Beispielen wurden ein kommerzieller Autoklav aus Edelstahl (Fa. Carl Roth) mit einem Volumen von 200 ml verwendet. Der Autoklav war bis 100 bar Arbeitsdruck und 300°C Arbeitstemperatur ausgelegt. Die gewünschte Betriebstemperatur wurde mit einem durch einen elektrischen Regler konstant gehaltenen Heizmantel eingestellt. Die Gase (Luft, Sauerstoff) wurden einer Stahlflasche über eine Druckarmatur entnommen. Dazu wurden mit Stahlgewebe ummantelte Rohrleitungen aus Teflon verwendet. Der gewünschte Arbeitsdruck wurde mit Hilfe von Ventilen eines Anzeigemanometers eingestellt. Um den Betriebsdruck von 100 bar nicht zu überschreiten, wurde zwischen dem Autoklaven und der Stahlflasche zusätzlich ein Sicherheitsventil, das auf 95 bar eingestellt war, eingebaut. Die Reaktionslösung wurde in einem Becherglas mit einem Volumen von 40 ml in den Autoklaven eingesetzt und von außen mit einem Rührmagnet gerührt. Die Drehzahl des Rührers gestattete eine feine Verteilung des Gases in der Reaktionslösung. Die Reinigung des Autoklaven wurde in kaltem Zustand mit Ethanol durchgeführt. Der Autoklav und das Entspannventil wurden vor jedem Versuch mit trockenem und reinem Stickstoff ausgeblasen. Glasgefäße wurden ferner in einem Ultraschallbad gereinigt. Nach dem Verschließen des Autoklaven wurde das oxidierende Gas (Luft oder Sauerstoff) aus einer Stahlflasche bis zum gewünschten Arbeitsdruck aufgedrückt. Man schloß dann das Einlaßventil, so daß eine weitere Gaszufuhr nicht mehr möglich war. Es wurde dann der Magnetrührer eingeschaltet und der Autoklaveninhalt auf die gewünschte Temperatur erhitzt. Nach Ablauf der Reaktionszeit wurde der Autoklav aus dem Heizmantel herausgenommen und in Eiswasser abgekühlt. Nach Abkühlen wurde dann das Glasgefäß mit dem Inhalt herausgenommen und analysiert.

### Reaktionsmedien

Als Reaktionsmedien wurden Mikroemulsionen (ME) verwendet, die zusammen mit den zu oxidierenden Verbindungen (Cyclopenten bzw. Cyclohexen) und Katalysatoren gebildet wurden. Die Zusammensetzungen dieser ME sind in Tabelle 1 aufgeführt.

**Tabelle 1**

| Mikroemulsionen (ME), Summe 100 Gew.-% ohne Katalysator | | | | | | | |
|---|---|---|---|---|---|---|---|
| Zusammensetzung [Gew.-%] | ME 1 | ME 2 | ME 3 | ME 4 | ME 5 | ME 6 | ME 7 |
| Cyclopenten | 39,3 | 32,1 | 32,1 | 30,6 | - | - | - |
| Cyclohexen | - | - | - | - | 30,2 | 30,2 | 30,2 |
| n-Heptan | 39,3 | 32,1 | 32,1 | 30,6 | - | - | - |
| n-Octan | - | - | - | - | 30,2 | 30,2 | 30,2 |
| Igepal* | 10,1 | 8,3 | 8,3 | 11,8 | - | - | - |
| Triton** | 3,5 | 6,7 | 6,7 | 5,8 | 18,8 | 18,8 | 18,8 |
| n-Propanol | - | 14,4 | 14,4 | 14,2 | 14,8 | 14,8 | 14,8 |
| Wasser | 7,8 | 6,4 | - | 7 | 6 | - | - |
| 1n Salzsäure | - | - | 6,4 | - | - | 6 | - |
| 0,5 n Natriumacetat | - | - | - | - | - | - | 6 |

| | | | | | | | |
|---|---|---|---|---|---|---|---|
| * Igepal CA 520: 4-(C₈H₁₇)C₆H₄O(CH₂CH₂O)₄CH₂CH₂OH (Fa. Aldrich) | | | | | | | |
| ** Triton X 100: 4-(C₈H₁₇)C₆H₄O(CH₂CH₂O)₉CH₂CH₂OH (Fa. Aldrich) | | | | | | | |

### Analyse

Bei der Durchführung der nachstehenden Beispiele wurde die Identität der erhaltenen Verbindungen durch gaschromatographische und massenspektroskopische Analyse im Vergleich mit authentischen Proben sichergestellt. Verwendet wurde ein Gaschromatograph HP 5890 Serie II mit einem Flammenionisationsdetektor und automatischem Probengeber. Als Trennsäulen dienten Kapillarsäulen OV 1701. Die Länge bzw. der Innendurchmesser der Säulen betrugen 25/50 m bzw. 0,32 mm. Der Einspritzblock und der Detektor wurden auf 300°C eingestellt. Die Temperatur der Säule wurde von 50°C mit 10°C/min Aufheizgeschwindigkeit auf 250°C gesteigert. Als Trägergas wurde Helium verwendet. Die Strömungsgeschwindigkeit des Heliums betrug 1,5 ml/min. Die quantitative Bestimmung erfolgte nach der Methode des inneren Standards. Als Standard wurde n-Nonan, bzw. n-Decan verwendet. Die Substanzen wurden mit Hilfe eines massenselektiven Detektors identifiziert.

### Beispiele 1 bis 19

In den vorstehend beschriebenen Rührautoklaven wurden jeweils ein Ansatz von 17 g (ca. 20 ml) mit einem Gehalt des in den nachstehenden Tabellen 2 und 3 angegebenen Katalysators in einem 40 ml Glasgefäß eingebracht und bei den in diesen Tabellen angegebenen Temperaturen und Sauerstoffdrücken während der angegebenen Zeit oxidiert. Anschließend wurde das Reaktionsprodukt gaschromatographisch und massenspektroskopisch analysiert sowie, wie weiter vorn beschrieben, aus der ME reines Produkt abgetrennt. Die nach der gaschromatographischen Analyse berechneten Umsätze und Selektivitäten sind ebenfalls in den Tabellen 2 und 3 angegeben.

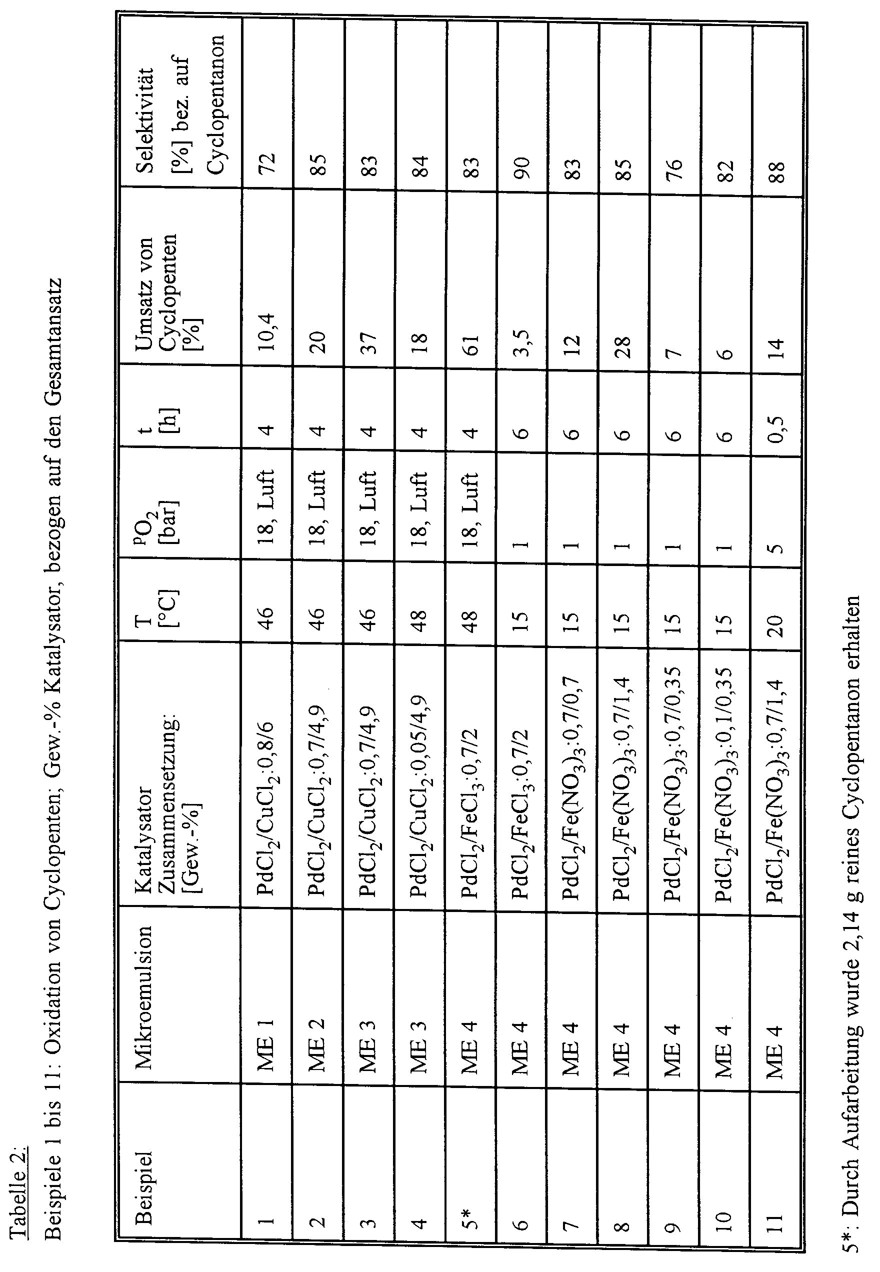

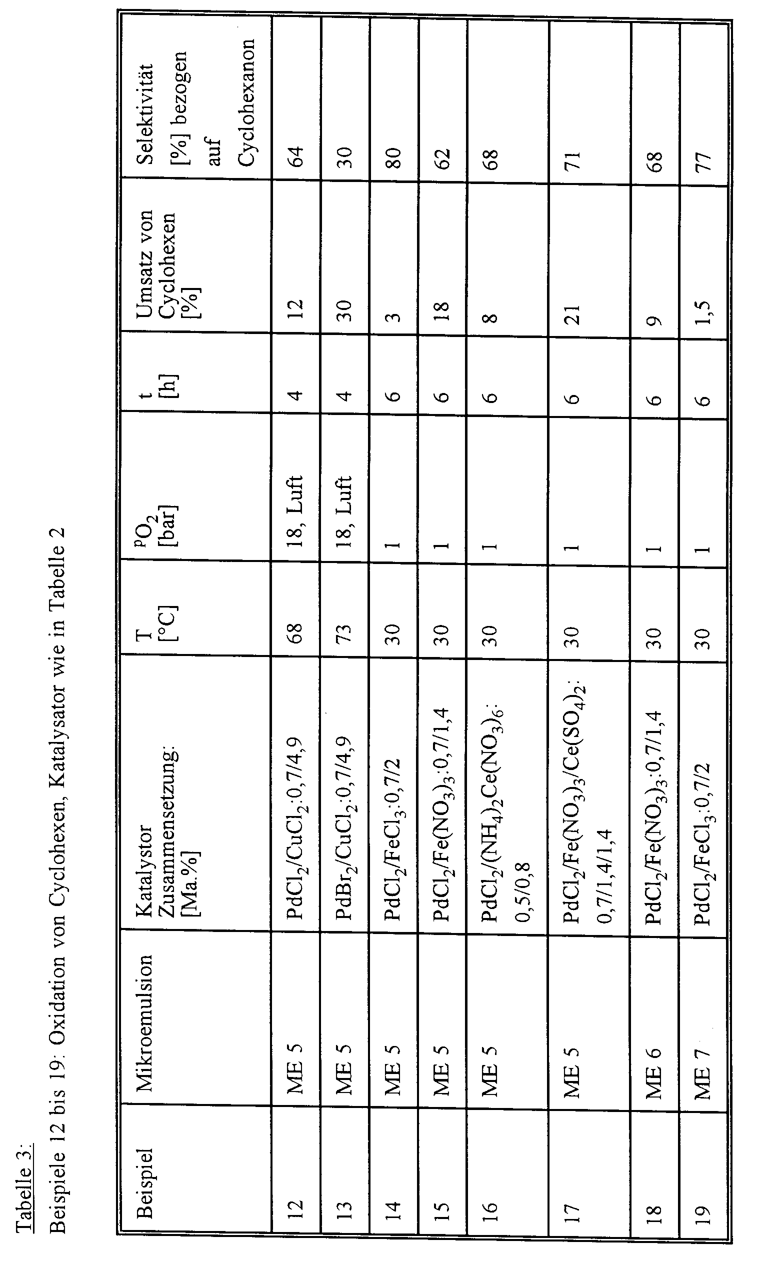

### Beispiel 20: Oxidation von 1-Octen

In den vorstehend beschriebenen Autoklaven wurden 17 g mit 1-Octen als zu oxidierender Verbindung gebildete ME folgender Zusammensetzung eingefüllt (in Gew.-% einschließlich Katalysator): 54 % 1-Octen, 7,7 % Igepal, 1,9 % Triton 100, 19,2 % n-Propanol, 10,7 % Wasser und 0,8 % PdCl₂/5,7 % Fe(NO₃)₃ als Katalysator. Die Oxidation wurde 6 h lang bei 50°C und 1 bar mit reinem Sauerstoff durchgeführt. Als Produkt wurde Methylhexylketon erhalten. Die Selektivität betrug 54 % bei einem Umsatz von 14 %.

### Beispiel 21: Oxidation von Ethen

Für die Oxidation von Ethen wurde die vorstehend beschriebene Apparatur verwendet, jedoch wurde durch den Autoklaven ein Gasstrom mit Hilfe eines Ventilsystems (Gaszuführung und Gasabführung) bei konstantem Druck mit konstanter Strömungsgeschwindigkeit aufrechterhalten. Zur Durchführung der Oxidation wurden in den Autoklaven 50 g ME, bestehend aus 39,37 g n-Heptan, 4,5 g Igepal, 5 g Wasser, 1 g 40 %ige wäßrige Fe(NO₃)₃-Lösung und 0,13 g PdCl₂ eingefüllt und ein Gasgemisch aus 85 Vol.-% Ethen und 15 Vol.-% Sauerstoff bei 12 bar und 24°C durch die ME geleitet. Die Strömungsgeschwindigkeit betrug 0,8 l/h. Die ME diente hier als Katalysatorträger. Im austretendem Gasgemisch wurden gaschromatographisch Acetaldehyd und Ethylenoxid als Produkte gefunden. In stationärem Zustand betrug der Umsatz 1,4 % des Ethens bzw. die Selektivität (bezogen auf Acetaldehyd) 62 %.

### Beispiel 22: Oxidation von Ethen

Es wurde, wie im Beispiel 21 beschrieben, gearbeitet, jedoch wurde ein Gasstrom aus 98 Vol.-% Sauerstoff und 2 Vol.% Ethen eingestellt. Dabei wurde ein Umsatz von 14 % des Ethens erzielt. Die Selektivität, bezogen auf Acetaldehyd, betrug 54 %.

### Beispiel 23: Oxidation von Propen

Es wurde, wie im Beispiel 21 beschrieben, gearbeitet, jedoch wurde ein Gasstrom aus 42 Vol.-% Sauerstoff und 48 Vol-% Propen eingestellt und bei 2 bar Gesamtdruck und Raumtemperatur mit einer Volumengeschwindigkeit von 0,8 l/h durch die ME geleitet. Die Selektivität für das Produkt Aceton betrug 64 %; als weiteres Produkt wurde Propylenoxid gefunden. Der Umsatz betrug 0,9 % des Propens.

## Patentansprüche

1. Verfahren zur Herstellung von Carbonylverbindungen der Formel 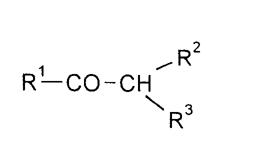 durch katalysierte Oxidation von Olefinen der Formel 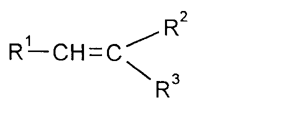 wobei in den Formeln
R¹, R² und R³ unabhängig voneinander Wasserstoff; geradkettiges oder verzweigtes C₁-C₈-Alkyl, das nicht substituiert oder 1-2fach durch Halogen, Hydroxy, Cyano, COO-C₁-C₄-Alkyl, CO-C₁-C₄-Alkyl, SO₂-O-C₁-C₂-Alkyl oder Phenyl substituiert ist; oder Phenyl oder Naphthyl, die nicht substituiert oder 1-2fach durch Halogen, Hydroxy, Nitro, Cyano, C₁-C₄-Alkyl, COO-C₁-C₄-Alkyl, CO-C₁-C₄-Alkyl oder SO₂-O-C₁-C₄-Alkyl substituiert sind, bedeuten,
und
R¹ und R³ oder R² und R³ weiterhin gemeinsam eine Alkylenkette (̵CH₂)̵ₘ mit m = 3-10 bilden können und 1-2 der Alkylenketten-C-Atome durch Halogen, Hydroxy, C₁-C₄-Alkyl, COO-C₁-C₄-Alkyl, CO-C₁-C₄-Alkyl, SO₂-O-C₁-C₄-Alkyl oder Phenyl substituiert sein können,
mit Sauerstoff oder sauerstoffhaltigen Gasen, dadurch gekennzeichnet, daß man die Oxidation bei 0-200°C, bevorzugt 0 bis 120°C, besonders bevorzugt 15 bis 80°C, ganz besonders bevorzugt 15 bis 40°C, und 0,2-200 bar, bevorzugt 0,5 bis 100 bar, besonders bevorzugt 1 bis 10 bar, in Gegenwart eines Katalysatorsystems, bestehend aus einer Palladiumverbindung und einer oder mehrerer Reoxidationsmittel aus der Gruppe von Verbindungen von weiteren Nebengruppenmetallen und Chinonen, in einer Mikroemulsion als Reaktionsmedium und Katalysatorträger durchführt.

2. Verfahren nach Anspruch 1, dadurch gekennzeichnet, daß als Mikroemulsion ein einphasiges System aus (i) 1-20 Gew.-% Wasser, (ii) 60-97 Gew.-% an einer oder mehreren organischen Komponenten aus der Gruppe der umzusetzenden Olefine und organischen Lösungsmitteln und (iii) 2-20 Gew.-% an einem oder mehreren Tensiden, bevorzugt 1-3 Tensiden, aus der Gruppe der ionischen und nichtionische Tenside eingesetzt wird, wobei alle Angaben auf das Gesamtgewicht der Mikroemulsion bezogen sind.

3. Verfahren nach Anspruch 2, dadurch gekennzeichnet, daß als Tenside solche aus der Gruppe der n-Alkyl(C₈-C₁₈)-sulfonate, n-Alkyl(C₈-C₁₈)-benzolsulfonate, n-Alkyl(C₈-C₁₈)-trimethyl-ammoniumsalze, Di-(n-alkyl(C₈-C₁₈))-dimethyl-ammoniumsalze, n-Alkyl(C₈-C₁₈)-carboxylate, Oligoethylenoxid(EO₂₋₃₀)-mono-n-alkyl(C₆-C₁₈)-ether, n-Alkyl(C₈-C₁₈)-dimethyl-aminoxide, n-Alkyl(C₈-C₁₈)-dimethyl-phosphinoxide oder Oligoethylenoxid(EO₂₋₃₀)-mono-arylether eingesetzt werden.

4. Verfahren nach Anspruch 1, dadurch gekennzeichnet, daß als Reoxidationsmittel eines oder mehrere Nebengruppenmetalle aus der Gruppe von Eisen, Kobalt, Nickel, Kupfer, Chrom, Zinn, Antimon, Cer, Rhodium, Platin, Gold, bevorzugt Eisen oder Kupfer, eingesetzt werden.

5. Verfahren nach Anspruch 4, dadurch gekennzeichnet, daß der Katalysator PdCl₂ oder Pd(NO₃)₂ und Fe(NO₃)₃ oder CuCl₂ enthält.

6. Verfahren nach Anspruch 1, dadurch gekennzeichnet, daß die Pd-Verbindung des Katalysators 0,0005-10 Gew.-% und die Nebengruppenmetallverbindungen 0,0001-20 Gew.-% der Mikroemulsion betragen.

7. Verfahren nach Anspruch 6, dadurch gekennzeichnet, daß die Pd-Verbindung 0,05-1 Gew.-% der Mikroemulsion und die Nebengruppenmetallverbindung das 2-100 fache der Pd-Verbindung betragen.

8. Verfahren nach Anspruch 1, dadurch gekennzeichnet, daß Olefine der Formel 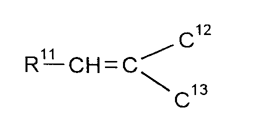 eingesetzt werden, in der
R¹¹ und R¹² unabhängig voneinander Wasserstoff; geradkettiges oder verzweigtes C₁-C₄-Alkyl, das nicht substituiert oder durch Halogen, Hydroxy, COO-CH₃, CO-CH₃ oder Phenyl substituiert ist; oder Phenyl, das nicht substituiert oder durch Halogen, Hydroxy, Nitro, CH₃, C₂H₅, COO-CH₃ oder CO-CH₃ substituiert ist und
R¹³ Wasserstoff oder Methyl bedeuten,
und R¹¹ und R¹³ oder R¹² und R¹³ gemeinsam Trimethylen, Tetramethylen oder Pentamethylen darstellen können.

9. In Mikroemulsionen angeordnete Katalysatoren, gekennzeichnet durch einen Gehalt von 0,0005-10 Gew.-% einer Pd-Verbindung und 0,0001-20 Gew.-% eines oder mehrerer Co-Katalysatoren, bezogen auf das Gesamtgewicht der Mikroemulsion.

10. Katalysatoren nach Anspruch 9, gekennzeichnet durch eine Konzentration des Co-Katalysators die das 2- bis 100fache der Konzentration der Pd-Verbindung beträgt.
